Europäisches Patentamt

European Patent Office

Office européen des brevets

(11) Veröffentlichungsnummer: **0 103 290**
**A2**

(12) # EUROPÄISCHE PATENTANMELDUNG

(21) Anmeldenummer: 83108953.7

(22) Anmeldetag: **10.09.83**

(51) Int. Cl.³: **A 61 K 31/765**, A 61 K 31/77, A 61 M 31/00, A 61 K 9/08

(30) Priorität: **14.09.82 DE 3234084**

(43) Veröffentlichungstag der Anmeldung: **21.03.84**
**Patentblatt 84/12**

(84) Benannte Vertragsstaaten: **AT BE CH DE FR GB IT LI LU NL SE** .

(71) Anmelder: **INTERMEDICAT GMBH, Gerliswilstrasse 43, CH-6020 Emmenbrücke (CH)**

(72) Erfinder: **Feller, Wolfgang, Dr., Ernstbergstrasse 30, D-3508 Melsungen (DE)**
Erfinder: **Schröder, Jürgen, Dr., Auf dem Rottheil 7, D-3509 Spangenberg (DE)**
Erfinder: **Werner, Heinz Helmut, Dr., Am Steig 2, D-3508 Melsungen (DE)**

(74) Vertreter: **von Kreisler, Alek, Dipl.-Chem. et al, Deichmannhaus am Hauptbahnhof, D-5000 Köln 1 (DE)**

(54) **Pharmazeutische Zubereitungen zur Behandlung von unerwünschten Verwachsungen sowie deren Verwendung.**

(57) Die Erfindung betrifft pharmazeutische Zubereitungen zur Behandlung von unerwünschten Verwachsungen, die dadurch gekennzeichnet sind, daß sie als Wirkstoff Verbindungen auf der Basis von Polyethylenglykol und/oder Polypropylenglykol oder deren Mischpolymerisate zusammen mit üblichen physiologisch verträglichen Trägern und Hilfsstoffen enthalten, sowie die Verwendung dieser pharmazeutischen Zubereitungen.

EP 0 103 290 A2

- 1 -

B.Braun Melsungen Aktiengesellschaft
Melsungen

Pharmazeutische Zubereitungen zur Behandlung
von unerwünschten Verwachsungen
sowie deren Verwendung

---

Die Erfindung betrifft pharmazeutische Zubereitungen zur Behandlung von unerwünschten Verwachsungen sowie die Verwendung dieser
bei der Bekämpfung von unerwünschten Verwachsungen.

Nach operativen Eingriffen in den Körperhöhlen oder nach entzündlichen Erkrankungen, die Oberflächen von Organen betreffen,
kommt es häufig zu unerwünschten Verwachsungen, die Ausgangspunkt späterer Beschwerden oder Zweiterkrankungen sein können.
Verwachsungen im Bauchraum können beispielsweise zu einem lebensbedrohlichen mechanischen Darmverschluß führen. Verwachsungen in der Region des Gleitgewebes an Sehnen können die Beweglichkeit von Gliedmaßen beeinträchtigen oder aufheben. Die
Verwachsung zwischen dem äußeren und inneren Blatt des Rippenfells führt zur Fixierung der Lunge an der Wand des Brustkörbs
mit einer entsprechenden Einschränkung der Atmungskapazität.

- 2 -

0103290

Die Oberfläche von Organen ist regelmäßig mit einer anatomisch variabel ausgebildeten Zellschicht bedeckt. Eine Zerstörung oder Schädigung dieser Oberflächen setzt nachfolgend beschriebene Heilungsvorgänge in Gang, die entweder zu einer Wiederherstellung des ursprünglichen Zustandes oder zu verschieden fester Verbindung benachbarter Organstrukturen führen. Auslösende Ursachen für diesen Vorgang können z.B. sein: Berührung mit Handschuhen oder chirurgischen Instrumenten, Kontakt mit Blut, Eiter, Bakterien, Benetzung durch Pharmaka mit vom physiologischen Wert abweichendem pH-Wert oder falscher Osmolarität, Austrocknung durch Wasserverlust während operativer Eingriffe.

Der Mechanismus der Verwachsung folgt den bekannten Vorgängen der Wundheilung, die in dem folgenden Schema dargestellt sind:

Fibrinogen
Gewebeschädigung → Fibrin → stab.Fibrin → Bindegewebe
Thrombin →

F XIII          Fibroblasten
Fibronectin     Kapillaren

In diesem Schema wird deutlich, daß unabhängig von der Art der auslösenden Ursache die Funktionsproteine Fibrinogen und Thrombin am Ende der Gerinnungskaskade notwendig sind, um die Bildung einer Verwachsung einzuleiten. Durch die Reaktion dieser beiden wasserlöslichen Komponenten entsteht unlösliches Fibrin als vorläufige Verklebung, die entweder durch Abbauprozesse wieder gelöst wird (Fibrinolyse), oder durch weitere Reaktion mit Faktor 13 und Einwachsen von Zellen mit spezifischer Syntheseleistung zur bindegewebigen Narbe ausreift.

In den vergangenen Jahrzehnten hat es vielfach Versuche gegeben, durch Verwendung bestimmter Pharmaka Verwachsungen nach operativen Eingriffen zu verhindern. Am bekanntesten ist die Verwendung von Polyvinylpyrrolidon-Lösungen, Dextranlösungen und Spülungen des Bauchraumes mit Elektrolyt-Lösungen.

Als Literaturbeispiele seien genannt:

A. Badowski, Z. Daciewicz, The importance of highmolecular weight colloid substances in the prevention of experimental peritoneal adhesions, Polish Medical Journal, Vol. X No. 1/1971; H. Bostedt, H.P. Brummer, Versuche zur Verhütung intraabdomineller Adhäsion nach geburtshilflichen Laparotomien beim Rind mit Hilfe von Polyvinylpyrrolidon, Tierärztl. Wochenschrift, 82.Jahrg., Heft 22, 15.11.1969; M.K. Mazuji, K. Kalambaheti, B. Pawar, Prevention of Adhesions with Polyvinylpyrrolidone, Archives of Surgery, Vol. 89, Dec. 1964; O. Kalligiannis, Postoperative Adhäsionsprophylaxe durch Kollidone, Klin.Chirurgie, 202, 6 (1961); H. Oettel, H. Rothländer, W. Schulze, Bericht über ein neues Verfahren zur Verhütung von Bauchfellverwachsungen durch Kollidon, Fortschritte der Medizin, Jg. 72, 1954, Heft 7/8; D. Carbase, C. Regnier, J. Berthet, A. Watrelot, C. Racinet, Prévention des adhérences péritonéales, J.Gyn.Obst.Biol.Repr., 1981, 10, 425 - 429.

Über den Wirkungsmechanismus der Verwachsungesprophylaxe wird in diesen Literaturangaben nichts ausgesagt. Die Erfolge waren nicht immer gut. Bei Verwendung von Polyvinylpyrrolidon besteht der Nachteil der Speicherung im Gewebe und der fehlenden Abbaumöglichkeit. Diese Lösungen sind daher aus dem Handel gezogen worden.

Zusammenfassend muß festgestellt werden, daß die gegenwärtigen Therapiemöglichkeiten zur Verwachsungsprophylaxe unbefriedigend geblieben sind.

Die Erfindung stellt sich die Aufgabe, eine pharmazeutische Zusammensetzung zur Verfügung zu stellen, die die Nachteile des Standes der Technik nicht aufweist und Verwachsungen erfolgreich verhindern kann.

Gelöst wird diese Aufgabe durch die Zurverfügungstellung von pharmazeutischen Zubereitungen zur Behandlung von unerwünschten Verwachsungen.

Dementsprechend betrifft die vorliegende Erfindung pharmazeutische Zubereitungen zur Behandlung von unerwünschten Verwachsungen, die dadurch gekennzeichnet sind, daß sie als Wirkstoff Verbindungen auf der Basis von Polyethylenglykol und/oder Polypropylenglykol oder Mischpolymerisate dieser Komponenten zusammen mit üblichen verträglichen Trägern und Hilfsstoffen enthalten.

Die Erfindung betrifft ferner die Verwendung von Polyethylenglykol und/oder Polypropylenglykol oder deren Mischpolymerisate enthaltenden Zubereitungen bei der Bekämpfung von unerwünschten Verwachsungen.

Die erfindungsgemäß verwendeten Polyethylenglykole werden auch als Polyethylenoxid oder Polyoxyethylen bezeichnet. Polyethylen-Polypropylenglykole sind beispielsweise unter dem Handelsnamen "Pluronics" im Handel erhältlich.

0103290

Das Molekulargewicht der erfindungsgemäß verwendeten Polyethylenglykole und/oder Polypropylenglykole liegt im Bereich von 400 bis 20 000, vorzugsweise im Bereich von 2 000 bis 6 000 und insbesondere bei 4 000. Besonders bevorzugt wird ein Polyethylenglykol (PEG) 4000, d.h. vom Molekulargewicht 4 000. Die pharmazeutischen Zubereitungen gemäß vorliegender Erfindung enthalten die Wirkstoffe, d.h. die Polyethylenglykole und/oder Polypropylenglykole in einer Konzentration von 0,5 bis 20 %, vorzugsweise 4 bis 15 %. Besonders bevorzugt sind beispielsweise Konzentrationen von 5 % und 10 %.

Die pharmazeutischen Zubereitungen gemäß der Erfindung können in den üblichen galenischen Zubereitungsarten vorliegen wie Formulierungen für die parenterale Applikation, z.B. Injektionslösungen, Spüllösungen oder in Formulierungen zur Implantation, beispielsweise als Depot-Präparate.

Als erfindungsgemäß verwendbare Lösungen der Polyethylenglykole und/oder Polypropylenglykole eignen sich z.B. wässrige Lösungen, physiologische Kochsalzlösungen, Ringer-Lösungen, glukosehaltige Lösungen sowie wässrige Lösungen, die durch Zusatz von Elektrolyten und Puffern auf einen physiologisch verträglichen pH-Wert gebracht worden sind. Bevorzugt werden pH-Bereiche von 6 bis 8. Der Zusatz von Elektrolyten und Puffern ermöglicht auch die Einstellung der Osmolarität in physiologisch gut verträgliche Bereiche. Ein bevorzugter Bereich der Osmolarität liegt bei 250 bis 350 mosm.

Bei tierexperimentellen Untersuchungen im Bauchraum von Tieren zur Ermittlung toxikologischer Daten zeigte sich überraschend, daß bereits ein verhältnismäßig

- 6 -

010 3 290

geringer Zusatz von Polyethylenglykol ausreichend war, um die sonst regelmäßg auftretenden Verwachsungen bei überlebenden Tieren vollständig zu verhindern. Es ist völlig überraschend, daß die Instillation von Polyethylenglykol enthaltenden Lösungen in Körperhöhlen unerwünschte Verwachsungen sicher verhindern kann.

Die Erfindung wird durch die nachfolgenden Beispiele und Versuche weiter erläutert.

Beispiel 1

10 g Polyethylenglykol vom Molekulargewicht 4 000 (PEG 4000) wurden in 1 Liter bidestilliertem Wasser gelöst. Nach Abfüllen in Injektionsampullen wurden die Lösungen nach bekanntem Verfahren hitzesterilisiert.

Beispiel 2

In 1 000 ml Polyvinylpyrrolidon (PVP)-Jod-Lösung wurden 250 g Polyethylenglykol vom Molekulargewicht 4000 gelöst und in Flaschen abgefüllt. Diese Lösungen sind autosteril.

Beispiel 3

In 1 000 ml einer erwärmten 10 %igen Gelatinelösung wurden 200 g Polypropylenglykol vom Molekulargewicht 2000 gelöst. Nach Zusatz von 1 g konzentrierter Formaldehydlösung wurde das Gemisch schnell in Formen von 50 ml Volumen gegossen und erstarrte durch Abkühlen und Vernetzung der Gelatine. Die erhaltenen Formkörper sind als Implantate geeignet. Sie wurden nach Einschweißen in Polyethylenfolien durch Gammabestrahlung mit einer Dosis von 2,5 Mrad sterilisiert.

Die folgenden Tierversuche wurden durchgeführt:

## Versuch 1

5 Wistar-Ratten, männlich, in der Gewichtsklasse von 150 bis 200 g, erhielten in Äthernarkose die unter Beispiel 2 beschriebene Lösung intraperitoneal injiziert. 5 weitere Tiere erhielten als Kontrolle eine 1%ige PVP-Jod-Lösung ohne PEG-Zusatz. Die Tiere wurden nach 14 Tagen getötet. Bei der Inspektion des Bauchraumes zeigte sich, daß alle Tiere mit der Prüflösung nach Beispiel 2 keine Verwachsungen im Bauchraum aufwiesen, während die Kontrolltiere Verwachsungsstränge und flächige Verklebungen zwischen verschiedenen Bauchorganen hatten. Die Leberoberfläche der Kontrolltiere war von einer derben bindegewebigen Kapsel bedeckt.

## Versuch 2

10 Wistar-Ratten der Gewichtsklasse zwischen 200 und 250 g wurden in Nembutal-Narkose durch intraperitoneale Injektion von 5 ml 0,05 n HCl eine Säureverätzung des Bauchfelles zugeführt. Die Säure wurde nach 5 Minuten Einwirkzeit durch eine äquivalente Menge NaOH neutralisiert. Die Hälfte der Tiere erhielt 5 ml physiologische Kochsalzlösung als Kontrolle, die andere Hälfte wurde mit einer Lösung nach Beispiel 1 durch intraperitoneale Injektion behandelt. Auch hier zeigt eine Kontrolle 14 Tage nach Tötung der Tiere, daß die mit dem Wirkstoff behandelte Gruppe verwachsungsfrei ist, während die Kontrolle schwere Verwachsungen aufweist.

## Versuch 3

Zerkleinerte Implantatblöcke, hergestellt nach Beispiel 3, wurden in den Bauchraum von Kaninchen eingepflanzt. Das Bauchfell wurde durch mechanische Schädigung mit Schmirgelpapier der Körnung 120 aufgerauht, bis es zu leichten Blutungen kam. Auch hier zeigte der Befund nach 14 Tagen fehlende Verwachsungen bei Anwendung der Implantate im Vergleich zu entsprechenden Kontrolltieren, die Verwachsungen aufwiesen.

Versuch 4

Versuchsserie nach Versuch 2

10 Tiere getestet, Beurteilung nach 14 Tagen

| Testlösung | Anzahl der Tiere von 10 | | |
| --- | --- | --- | --- |
| | leichte | schwere | keine |
| | Verwachsungen | | |
| NaCl, 0,9 % | 2 | 8 | Ø |
| Ringerlösung | 3 | 7 | Ø |
| PEG 4000, 10 % | 1 | Ø | 9 |
| PEG 4000 5 % NaCl 0,45% | Ø | Ø | 10 |
| PEG 4000 1% NaCl 0,8% | 3 | Ø | 7 |
| Dextranlösung Mol.-Gew.6000, 6% | 3 | 6 | 1 |

0103290

Patentansprüche

1. Pharmazeutische Zubereitungen zur Behandlung von unerwünschten Verwachsungen, dadurch gekennzeichnet, daß sie als Wirkstoff Verbindungen auf der Basis von Polyethylenglykol und/oder Polypropylenglykol oder deren Mischpolymerisate zusammen mit üblichen physiologisch verträglichen Trägern und Hilfsstoffen enthalten.

2. Pharmazeutische Zubereitungen nach Anspruch 1, dadurch gekennzeichnet, daß die Wirkstoffe in einer Konzentration zwischen 0,5 und 20 % vorliegen.

3. Pharmazeutische Zubereitungen nach Ansprüchen 1 und 2, dadurch gekennzeichnet, daß die Wirkstoffe ein Molekulargewicht von 4 000 bis 20 000, vorzugsweise von 2 000 bis 6 000 aufweisen.

4. Pharmazeutische Zubereitungen nach Ansprüchen 1 bis 3, dadurch gekennzeichnet, daß sie in Form von wässrigen Lösungen vorliegen, deren pH-Wert und Osmolarität durch Zusatz von Elektrolyten und Puffern in einem Bereich von pH 6 - 8 und einem Bereich der Osmolarität von 250 - 350 mosm. eingestellt sind.

5. Verwendung von pharmazeutischen Zubereitungen, die als Wirkstoff Polyethylenglykole und/oder Polypropylenglykole oder deren Mischpolymerisate zusammen mit üblichen physiologisch verträglichen Trägern und Hilfsstoffen enthalten bei der Bekämpfung von unerwünschten Verwachsungen.

6. Verwendung nach Anspruch 5, dadurch gekennzeichnet, daß die Wirkstoffe in einer Konzentration zwischen 0,5 und 20 % vorliegen.

7. Verwendung nach Ansprüchen 5 und 6, dadurch gekennzeichnet, daß die Polyethylenglykole und/oder Polypropylenglykole oder deren Mischpolymerisate ein Molekulargewicht von 400 bis 20 000, vorzugsweise von 2 000 bis 6 000 aufweisen.

8. Verwendung nach Ansprüchen 5 - 7, dadurch gekennzeichnet, daß die Wirkstoffe in Form von wässrigen Lösungen vorliegen, die durch Zusatz von Elektrolyten und Puffern auf einen pH-Wert im Bereich von 6 - 8 und eine Osmolarität im Bereich von 250 bis 350 mosm. eingestellt worden ist.